# EUROPÄISCHE PATENTSCHRIFT

(11) **EP 1 066 241 B1**
(45) Veröffentlichungstag und Bekanntmachung des Hinweises auf die Patenterteilung: **18.08.2004**
(21) Anmeldenummer: 99916885.9
(22) Anmeldetag: 24.03.1999
(51) Int. Cl.: C07C 51/44, C07C 51/487, C07C 51/50, C07C 57/04, C07C 67/08, C07C 69/14, C07C 69/54

(54) **VERFAHREN ZUR HERSTELLUNG VON (METH)ACRYLSÄURE UND (METH)ACRYLSÄUREESTERN**
METHOD FOR PRODUCING (METH)ACRYLIC ACID AND (METH)ACRYLIC ACID ESTERS
PROCEDE DE PREPARATION D'ACIDE (METH)ACRYLIQUE ET D'ESTERS D'ACIDE (METH)ACRYLIQUE

(30) Priorität: 31.03.1998 DE 19814449
(43) Veröffentlichungstag der Anmeldung: 10.01.2001
(73) Patentinhaber: BASF AKTIENGESELLSCHAFT, 67056 Ludwigshafen (DE)
(72) Erfinder: AICHINGER, Heinrich, D-68199 Mannheim (DE); MARTAN, Hans, D-67227 Frankenthal (DE); NESTLER, Gerhard, A-1070 Wien (AU)
(74) Vertreter: Isenbruck, Günter, Dr.
(86) Internationale Anmeldenummer: PCT/EP1999/001997
(87) Internationale Veröffentlichungsnummer: WO 1999/050221

(56) Entgegenhaltungen:
- EP-A- 0 270 999
- EP-A- 0 648 732
- EP-A- 0 713 854
- EP-A- 0 727 408
- EP-A- 0 770 592
- DATABASE WPI Derwent Publications Ltd., London, GB; AN 1976-10272X XP002117604 & JP 50 117716 A (NIPPON KAYAKU KK), 16. September 1975 (1975-09-16)
- DATABASE WPI Derwent Publications Ltd., London, GB; AN 1980-83357C XP002117605 & JP 55 129239 A (MITSUBISHI RAYON CO LTD) , 6. Oktober 1980 (1980-10-06)

## Beschreibung

Die vorliegende Erfindung betrifft ein Verfahren zur Herstellung von Rein-(Meth)acrylsäure aus einer Roh-(Meth)acrylsäure, wobei eine wasserarme Roh-(Meth)acrylsäure, die neben (Meth)acrylsäure im wesentlichen noch Essigsäure und gegebenenfalls Aldehyde enthält, mit einer Verbindung, die in der Lage ist, Aldehyde zu entfernen, behandelt wird und die so erhaltene aldehydfreie Roh-(Meth)acrylsäure einer unscharfen Destillation unterworfen wird, und anschließend die als Sumpfprodukt anfallende (Meth)acrylsäure, die weitgehend essigsäurefrei ist, von den übrigen Hochsiedern abgetrennt wird und Rein-(Meth)acrylsäure erhalten wird. Vorzugsweise wird die bei der unscharfen Destillation erhaltene, hauptsächlich aus (Meth)acrylsäure und Essigsäure bestehende Leichtsiederfraktion mit Alkanolen verestert, wobei (Meth)acrylsäureester erhalten werden.

Unter dem erfindungsgemäß verwendeten Begriff "Roh-(Meth)acrylsäure" wird ein (meth)acrylsäurehaltiges Gemisch verstanden, das durch katalytische Gasphasenoxidation von C₃- bzw. C₄- Vorläufern, Absorption in einem hochsiedenden Lösungsmittel, Desorption der Leichtsieder und destillativer Abtrennung aus dem Lösungsmittel hergestellt wird, mindestens 90 Gew.-% (Meth)acrylsäure enthält und weitgehend wasserfrei ist.

Unter "Rein-(Meth)acrylsäure" wird eine aldehydfreie (Meth)acrylsäure mit einer Reinheit von mindestens 99,7 Gew.-% verstanden, die zur Herstellung von hochmolekularen Polymerisaten geeignet ist.

Der im Rahmen der vorliegenden Anmeldung verwendete Begriff (Meth)-acrylsäure(ester) steht für Acrylsäure und Methacrylsäure beziehungsweise deren Ester.

(Meth)acrylsäure bildet aufgrund ihrer sehr reaktionsfähigen Doppelbindung sowie der Säurefunktion ein wertvolles Monomeres zur Herstellung von Polymerisaten, z.B. für als Klebstoffe geeignete wäßrige Polymerisatdispersionen.

Unter anderem ist Acrylsäure zugänglich durch Gasphasenoxidation von Propylen und/oder Acrolein mit Sauerstoff oder Sauerstoff enthaltenden Gasen in Gegenwart von Katalysatoren bei erhöhter Temperatur, infolge der hohen Reaktionswärme vorzugsweise unter Verdünnen der Reaktionspartner mit Inertgasen und/oder Wasserdampf.

Als Katalysatoren werden dabei in der Regel oxidische Mehrkomponentensysteme, z.B. auf Basis von Molybdän-, Chrom-, Vanadium- oder Telluroxiden, eingesetzt.

Bei diesem Verfahren wird jedoch nicht reine Acrylsäure, sondern ein Gasgemisch, das neben Acrylsäure als Nebenkomponenten im wesentlichen nicht umgesetztes Acrolein und/oder Propylen, Wasserdampf, Kohlenoxide, Stickstoff, Sauerstoff, Essigsäure, Formaldehyd, Benzaldehyd, Furfurale und Maleinsäureanhydrid enthält, erhalten, von welchem die Acrylsäure anschließend abgetrennt werden muß.

Die Herstellung von Methacrylsäure ist ausgehend von den entsprechenden C₄-Verbindungen analog möglich.

Eine derartig verunreinigte (Meth)acrylsäure (Roh-(Meth)acrylsäure) kann nicht direkt z.B. zur Herstellung von hochmolekularen Polymeren weiterverwendet werden, sondern muß einer aufwendigen Reinigungsprozedur unterworfen werden. In der Regel sind dazu mehrere Destillationsschritte erforderlich, um die notwendige Reinheit von mindestens ungefähr 99,7 % (Rein-(Meth)acrylsäure) zu erreichen (s. Kirk-Othmer, Encycl. of Techn. Chem., 4. Aufl., S. 299-300).

Aldehyde, die das Polymerisationsverhalten der (Meth)acrylsäure negativ beeinflussen, werden in der Regel durch Behandlung mit primären Aminen, Hydrazinen oder Aminoguanidin und anschließender Destillation entfernt. Dabei erfolgt die Zugabe dieser Verbindungen, die in der Lage sind, Aldehyde zu entfernen, vor der Destillation.

Die destillative Abtrennung der Essigsäure von der (Meth)acrylsäure stellt aufgrund der geringen Siedepunktsunterschiede und der allgemein großen Polymerisationsneigung der (Meth)acrylsäure bei thermischer Belastung einen ausgesprochen schwierigen Reinigungsschritt dar, der einen hohen technischen Aufwand und Ausbeuteverluste verursacht.

Gemäß der DE-A 19 50 750 (s. Sp.2, Z.31-53) ist eine Destillationskolonne mit mindestens 55 Böden und ein Rücklaufverhältnis von 15 notwendig, um die Trennung von Acrylsäure und Essigsäure einigermaßen zu bewerkstelligen.

Zur Lösung dieses Problems wurden bislang verschiedene Vorschläge unterbreitet:

Die Abtrennung von Acrylsäure, z.B. aus dem Reaktionsgemisch der Propylenoxidation, das im wesentlichen aus Acrylsäure, Essigsäure, Aldehyden und Wasser besteht, mit Hilfe einer Azeotropdestillation, wobei die Essigsäure und das Wasser mit einem Schleppmittel abdestilliert werden; als Schleppmittel werden beispielsweise Ester, Alkohole, Ketone, Aromaten, Alkane oder Gemische davon eingesetzt (s. DE-A 19 50 750, GB-B 1 120 284 und EP-A 0 551 111).

Die JA 71-06 886 schlägt zur Lösung dieses Problems eine Extraktivdestillation mit Formamiden und/oder Acetamiden vor.

Die oben beschriebenen Verfahren sind technisch aufwendig. Ferner wird gemäß diesen Druckschriften die bei obigen Verfahren anfallende Leichtsiederfraktion, die hauptsächlich aus Essigsäure, Acrylsäure, Aldehyden, Wasser und gegebenenfalls Lösungsmittel besteht, in der Regel verworfen.

Die EP-A 0 727 408 schlägt die Verwendung der bei der destillativen Gewinnung von Rein-Acrylsäure anfallenden Leichtsiederfraktion zur Herstellung von Essigsäurealkylestem vor. Voraussetzung dafür ist eine möglichst scharfe Essigsäure-Acrylsäure-Trennung, was große Probleme und hohen technischen Aufwand mit sich bringt und eine aufwendige Reinigung des Esters erfordert. Das dort beschriebene Verfahren ist daher vergleichsweise unwirtschaftlich.

Die DE-A 21 64 767 schlägt eine destillative Trennung vor, bei der in der Leichtsiederfraktion ein Acrylsäure-Gehalt von 10 bis 70 % eingestellt wird. Auf diese Weise sollen die üblichen Polymerisationsprobleme weitgehend verhindert werden. Das Essigsäure-Acrylsäure-Gemisch wird der Aufarbeitung der wäßrigen Roh-Acrylsäure zugeführt. Dieses Verfahren ist nur dann durchführbar, wenn eine eigene Roh-Acrylsäure-Herstellung oder eine Einrichtung zur Aufarbeitung wäßriger Acrylsäurelösungen vorhanden sind, was lediglich in seltenen Fällen der Fall ist.

Auch die Herstellung von (Meth)acrylsäureestern durch säurekatalysierte Veresterung von (Meth)acrylsäure mit einem oder mehreren Alkanolen ist aus dem Stand der Technik bekannt. Bezüglich derartiger Veresterungsreaktionen ist ganz allgemein bekannt, daß es sich um Gleichgewichtsreaktionen handelt und von daher die Anwesenheit von Wasser im Reaktionsgleichgewicht wirtschaftliche Umsätze verhindert. Demgemäß wird in der Regel weitgehend wasserfreie (Meth)acrylsäure eingesetzt und das bei der Veresterung entstehende Reaktionswasser, gegebenenfalls mit Hilfe eines Schleppmittels, destillativ entfernt.

Wie bereits eingangs erwähnt, entstehen bei der Herstellung von (Meth)acrylsäure durch Oxidation ausgehend von den entsprechenden C₃-/C₄- Vorläufern noch beträchtliche Mengen an Essigsäure (ca. 0,5 bis 10 Gew.-%). Aufgrund der geringen Siedepunktsunterschiede und der hohen Polymerisationsneigung der (Meth)acrylsäure bei thermischer Belastung ist eine destillative Abtrennung der oben genannten Nebenprodukte schwierig und aufwendig (US 3 844 903, DE-A 2 164 767).

Die EP-A 0 713 854 beschreibt ein Verfahren zum Abtrennen von Aldehyden aus Roh-(Meth)acrylsäure, bei dem man die Roh-(Meth)acrylsäure mit einem Carbonsäurehydrazid und/oder dessen Salz versetzt und anschließend aus dem Gemisch die (Meth)acrylsäure destillativ abtrennt.

Aus EP-A 0 770 592 ist es bekannt, Rein-Acrylsäure mit einem Restgehalt an Aldehyden unter 10 ppm durch Destillation von Roh-Acrylsäure in Gegenwart von aus zwei Gruppen ausgewählten Aminen durchzuführen, wobei die erste Gruppe primäre Arylamine und die zweite Gruppe ortho-, meta-, para-Phenylendiamin, 4-Nitrophenylhydrazin und 2,4-Dinitrophenyl-Hydrazin umfasst.

Da bei der Veresterung von essigsäurehaltiger (Meth)acrylsäure mit Alkanolen auch die Essigsäure verestert wird, bedingt die Bildung von Essigsäureestem einen zusätzlichen Trennaufwand und einen Verlust an Alkanol. Dabei ist ferner zu beachten, dass die destillative Abtrennung des Essigsäureesters aus dem Veresterungsgemisch, vor allem die Trennung von nicht umgesetztem Alkanol, wegen der Ausbildung von binären Azeotropen, behindert wird.

Im Falle von z.B. Butanol siedet das Butanol-Butylacetat-Azeotrop bei 115,8°C (57% Butanol), wobei Butanol bei 117,4°C und Butylacetat bei 125,6°C sieden.

Da die Essigsäureester relativ leicht flüchtig und nicht polymerisierbar sind, werden bei der Herstellung von Polymerisaten in der Regel hochreine (Meth)acrylsäureester, d.h. (Meth)acrylsäureester, die möglichst, d.h. im wesentlichen, frei von Essigsäureestern sind, benötigt. Der beispielsweise in einer Anstrichdispersion oder in einem Klebstoff verbleibende Essigsäureester würde nämlich unter anderem eine starke Geruchsbelästigung hervorrufen. Eine aufwendige Entfernung (Desodorierung) des Essigsäureesters wäre notwendig.

Wie sich aus obigem ergibt, besteht bei der (Meth)acrylsäureester-Herstellung prinzipiell das Problem eines zu hohen Verbrauchs an Alkanolen, was sowohl wirtschaftlich als auch ökologisch nachteilig ist.

In Anbetracht des obigen Standes der Technik lag der vorliegenden Erfindung die Aufgabe zugrunde, ein technisch einfach durchführbares Verfahren zur Herstellung von Rein-(Meth)acrylsäure bereit zu stellen, in dem die bei der Herstellung von Rein-(meth)acrylsäure anfallenden weiteren Wertprodukte, falls dies erwünscht ist, ebenfalls vorteilhaft verwendet werden können.

Demgemäß betrifft die vorliegende Erfindung ein Verfahren zur Herstellung von Rein-(Meth)acrylsäure, ausgehend von einer Roh-(Meth)acrylsäure, die mindestens 0,2 bis 10 Gew.% Essigsäure, 0,05 bis 1 Gew.% Aldehyde und 0,05 bis 5 Gew.% Wasser enthält, das die folgenden Stufen umfaßt:
A: Behandlung der Roh-(Meth)acrylsäure mit mindestens einer Verbindung, die in der Lage ist, Aldehyde zu entfernen, wobei eine aldehydfreie Roh-(Meth)acrylsäure erhalten wird;
B: Unterwerfen der aldehydfreien Roh-(Meth)acrylsäure einer unscharfen Destillation, wobei eine (Meth)acrylsäure und Essigsäure enthaltende Leichtsiederfraktion und eine (Meth)acrylsäure und Hochsieder enthaltende Schwersiederfraktion erhalten wird; und
C: Abtrennung einer Rein-(Meth)acrylsäure aus der Schwersiederfraktion.
D: Veresterung der in Stufe B erhaltenen, (Meth)acrylsäure und Essigsäure enthaltenden Leichtsiederfraktion mittels einem oder mehreren Alkanolen, wobei ein Veresterungsgemisch umfassend einen oder mehrere (Meth)acrylsäureester, einen oder mehrere Essigsäureester und ein oder mehrere Alkanole erhalten wird.

Dabei wird im einzelnen wie folgt vorgegangen:

Eine durch Gasphasen-Oxidation und anschließende Aufarbeitung, wie in der Einleitung der vorliegenden Anmeldung erwähnt, erhaltene Roh-(Meth)acrylsäure, wird zunächst mit einer Verbindung, die in der Lage ist, Aldehyde zu entfernen, behandelt, wobei eine aldehydfreie bzw. im wesentlichen aldehydfreie Roh-(Meth)acrylsäure erhalten wird. Der Gehalt an Aldehyden in der aldehydfreien Roh-(Meth)acrylsäure beträgt im allgemeinen nicht mehr als 10 ppm, vorzugsweise nicht mehr als 5 ppm.

Im allgemeinen wird dazu die Roh-(Meth)acrylsäure bei 20 bis 40°C einer Behandlung mit einer Verbindung, die in der Lage ist, Aldehyde zu entfernen, vorzugsweise einer amingruppenhaltigen Verbindung, unterzogen. Im einzelnen sind dabei insbesondere Aminoguanidinhydrogencarbonat, Hydrazin oder Adipinsäuredihydrazid, sowie Gemische aus zwei oder mehr davon zu nennen.

Die oben beschriebene Behandlung dauert in der Regel 2 bis 20 Stunden.

Anschließend wird die aldehydfreie Roh-(Meth)acrylsäure einer unscharfen Destillation unterworfen, die in erster Linie dazu dient, die in der Roh-(Meth)acrylsäure noch vorhandene Essigsäure abzutrennen. Während dieser unscharfen Destillation wird eine Leichtsiederfraktion erhalten, die mindestens 80 Gew.-% (Meth)acrylsäure enthält.

Der erfindungsgemäß verwendete Begriff "unscharfe Destillation" bezeichnet eine Destillation, bei der ein Gemisch, das zwei oder mehr Komponenten enthält, unter solchen Bedingungen destilliert wird, daß lediglich eine erste Komponente (hier: (Meth)acrylsäure) in hoher Reinheit erhalten wird und die andere(n) Komponente(n) als Mischung mit der ersten Komponente und gegebenenfalls weiteren im Gemisch enthaltenen Bestandteilen (hier: Essigsäure und (Meth)acrylsäure) anfällt.

Die unscharfe Destillation wird in einer dem Fachmann allgemein bekannten Destillationseinheit durchgeführt. In der Regel wird eine Kolonne mit 20 bis 50 Böden, vorzugsweise Glocken-, Sieb- und Dual-Flow-Böden oder eine entsprechende Packungskolonne eingesetzt. Die Sumpftemperatur während der Destillation beträgt im allgemeinen 50 bis 130°C, vorzugsweise 60 bis 100°C, wobei bei einem Druck am Kolonnenkopf von 20 bis 200 mbar und einem Rücklaufverbältnis von 1 bis 10 gearbeitet wird.

Vorzugsweise wird bei einer Temperatur von 60 bis 100°C, einem Druck am Kolonnenkopf von 20 bis 200 mbar, einem Rücklaufverhältnis von 1 bis 10 unter Verwendung einer Kolonne mit Dual-Flow-Böden gearbeitet.

Die Stabilisierung der Kolonne erfolgt durch Zugabe eines Polymerisationsinhibitors zum Rücklauf, wobei vorzugsweise Phenothiazin, eine phenolische Verbindung, eine N-O-Verbindung, Kupfersalze, Sulfonsäuresalze oder ein Gemisch aus zwei oder mehr davon, weiter bevorzugt Phenothiazin oder Hydrochinon, ein Gemisch aus Phenothiazin und Hydrochinon, Hydrochinonmonomethylether, p-Nitrosophenol, Nitrosodiethylanilin oder Tetramethylpiperidin-1-oxylen, wie sie in der DE-A 1618 141 beschrieben sind, zugegeben.

Weiter bevorzugt erfolgt die Stabilisierung der Kolonne durch Verwendung von Sulfönsäuresalzen des Phenothiazins als Polymerisationsinhibitor. Diese Verbindungen können auf einfache Weise durch Umsetzung von Phenothiazin mit entsprechenden Sulfonsäuren gemäß der EP-A 0 775 686 erhalten werden. Besonders bevorzugt werden Thiodiphenyl-ammoniumalkylbenzolsulfonate, insbesondere solche mit C₆- bis C₂₀-Alkylgruppen, eingesetzt.

Somit betrifft die vorliegende Erfindung auch ein Verfahren worin eine Sulfonsäuresalzes des Phenothiazins als Polymerisationsinhibitor für (Meth)acrylsäure, verwendet wird.

Als Verdampfer beziehungsweise Kondensator werden ebenfalls die dem Fachmann bekannten Apparaturen eingesetzt, wobei vorzugsweise ein Robert-Verdampfer bzw. ein Quench-Kondensator, der bei 20 bis 40°C betrieben wird, Anwendung findet.

In Stufe B fällt neben der bereits diskutierten (Meth)acrylsäure und Essigsäure enthaltenden Leichtsiederfraktion auch eine (Meth)acrylsäure und Hochsieder enthaltende Schwersiederfraktion an. Diese als Sumpfprodukt anfallende Fraktion wird einer Abtrenn-Stufe C unterworfen, wobei mittels einer Destillation in dem Fachmann bekannter Weise Rein-(Meth)acrylsäure erhalten wird.

Dabei wird bei der Abtrennung der (Meth)acrylsäure durch Destillation Rein-(Meth)acrylsäure als Kopfprodukt gewonnen. Bei diesem Schritt wird keine große Trennarbeit geleistet, so daß die (Meth)acrylsäure vorzugsweise einfach "abgetoppt" werden kann.

Unter "Abtoppen" wird erfindungsgemäB eine Destillation verstanden, bei der das Produkt direkt über Kopf dampfförmig abgezogen und anschließend kondensiert wird und mit keinem Rücklauf in Kontakt tritt. Die dabei verwendete Kolonne hat praktisch keine trennwirksamen Einbauten.

Dieses "Abtoppen" erfolgt über einen Spritzschutz, wobei die Sumpftemperatur 50 bis 120°C, vorzugsweise 60 bis 100°C beträgt, und der Druck in Abhängigkeit von der verwendeten Sumpftemperatur entsprechend eingestellt wird.

Die Kondensation der Rein-(Meth)acrylsäure erfolgt mit Hilfe eines Quenches bei 20 bis 30°C.

Vorzugsweise wird der Spritzschutz mit reiner (Meth)acrylsäure beaufschlagt, die mit 150 bis 200 ppm Stabilisator, z.B. Hydrochinonmonomethylether stabilisiert wird.

Die Reinheit der erhaltenen Rein-(Meth)acrylsäure beträgt 99,7 bis 99,9 %, der Essigsäuregehalt 100 bis 800 ppm und der Furfuralgehalt maximal 1 ppm.

Die in Stufe B anfallende, hauptsächlich (Meth)acrylsäure und Essigsäure enthaltende Leichtsiederfraktion ist aldehydfrei bzw. im wesentlichen aldehydfrei und besteht aus mindestens 80 Gew.%, vorzugsweise, 80 bis 95 Gew.-% (Meth)acrylsäure, 3 bis 15 Gew.-% Essigsäure und 0,5 bis 10 Gew.-% Wasser. In das erfindungsgemäßen Verfahren wird die Leichtsiederfraktion auf bekannte Weise mit einem oder mehreren Alkanolen nach einem Verfahren gemäß des Standes der Technik, wie dies beispielsweise in der DE-A 195 47 485 und dem dort zitierten Stand der Technik beschrieben ist, verestert. Dabei wird die in Stufe (B) erhaltene Leicht-siederfraktion direkt mit einem C₁-C₁₂-, vorzugsweise C₁-C₁₀-Alkanol, insbesondere C₄-C₈-Alkanol verestert, wobei die genauen Veresterungsbedingungen vom verwendeten Alkanol abhängig sind.

Als bevorzugte Alkanole sind zu nennen:
Methanol, Ethanol, iso-Propanol, n-Propanol, n-Butanol, iso-Butanol, Octanol, 2-Ethylhexanol, vorzugsweise n-Butanol, iso-Butanol und 2-Ethylhexanol.

Typische Bedingungen, unter denen die Veresterung stattfinden kann, sind wie folgt:
Verhältnis Alkanol:(Meth)acrylsäure:
   1:0,7-1,2 (molar)
Katalysator:
   Schwefelsäure oder eine Sulfonsäure, wie z.B. p-Toluolsulfonsäure
Katalysatormenge:
   0,1 - 10 Gew.-% vorzugsweise 0,5 - 5 Gew.-%, jeweils bezogen auf die Einsatzstoffe
Stabilisator:
   Phenothiazin, Hydrochinon, Hydrochinonmonamethylether, Phenylendiamin und gegebenenfalls Luft
Stabilisatormenge:
   100 - 50.000 ppm, vorzugsweise 500 - 2.000 ppm, jeweils bezogen auf (Meth)acrylsäure.
Umsetzungstemperatur:
   80 - 160°C, vorzugsweise 90 - 130°C
Druck während der Umsetzung:
   0,5 bis 1,5 bar, vorzugsweise bei Atmosphärendruck
Umsetzungsdauer:
   1 bis 10 Stunden, vorzugsweise 1 bis 6 Stunden.

Gegebenenfalls kann ein Schleppmittel, wie z.B. Cyclohexan oder Toluol zur Entfernung des während der Veresterung entstehenden Wassers eingesetzt werden.

Die Veresterung an sich kann drucklos, mit Überdruck oder Unterdruck sowie kontinuierlich als auch diskontinuierlich durchgeführt werden, wobei eine kontinuierliche Führung des Gesamtverfahrens, d.h. eine kontinuierliche Durchführung der hierin erläuterten Stufen A bis F bevorzugt ist.

Da erfindungsgemäß die in Stufe B erhaltene Leichtsiederfraktion verestert wird, erhält man ein Veresterungsgemisch, das den/die gewünschten (Meth)acrylsäureester und darüber hinaus den/die entsprechenden Essigsäureester umfaßt. Ferner enthält das Veresterungsgemisch das oder die Alkanole.

Die Isolierung der (Meth)acrylsäureester erfolgt auf herkömmliche Weise. In der Regel werden dabei zunächst der Katalysator und die nicht umgesetzte (Meth)acrylsäure ausgewaschen und anschließend das Veresterungsgemisch aufgetrennt, vorzugsweise destillativ aufgetrennt.

Bei dieser Auftrennung wird einerseits der eine oder mehrere (Meth)acrylsäureester (im Sumpf) und andererseits ein Auftrennungsgemisch, das den/die Essigsäureester und ein oder mehrere Alkanole umfaßt, erhalten (Stufe E). Dieses Auftrennungsgemisch wird vorzugsweise in einer weiteren Stufe F verseift, wobei ein Verseifungsgemisch erhalten wird, das den einen oder mehrere Alkanole und Essigsäuresalze enthält. Aus diesem Verseifungsgemisch kann anschließend das Alkanol wiederum abgetrennt werden.

Im einzelnen wird dabei vorzugsweise wie folgt vorgegangen:

Das erhaltene Veresterungsgemisch wird vorzugsweise zwecks Abtrennung der Säure (Katalysator und nicht umgesetzter Carbonsäuren) mit wäßriger Laugenlösung, vorzugsweise 1 bis 10 Gew.-%-iger Natronlauge und Wasser gewaschen.

Aus dem weitgehend säurefreien Veresterungsgemisch werden in einer üblichen Destillationseinheit eine Leichtsiederfraktion, die u.a. den Essigsäureester, sowie nicht umgesetztes Alkanol umfaßt und ein Sumpfprodukt, das die Hauptmenge des (Meth)acrylsäureesters enthält, erhalten.

In der Regel wird dazu eine Destillationskolonne mit 40 bis 60 Böden, vorzugsweise Glocken-, Sieb- oder Dual-Flow-Böden, oder eine entsprechende Packungskolonne eingesetzt.

Die Sumpftemperatur beträgt dabei in Abhängigkeit vom eingesetzten Alkanol 50 bis 150°C, wobei der Druck entsprechend eingestellt wird. Das Rücklaufverhältnis beträgt im allgemeinen 3 bis 10. Die Stabilisierung der Kolonne erfolgt über den Rücklauf durch Zugabe von 50 bis 500 ppm Inhibitor, vorzugsweise Phenothiazin, Hydrochinon, Hydrochinonmonomethylether, Phenylendiamine, Nitrosoverbindungen oder Gemischen davon.

Die Menge der ausgeschleusten Leichtsiederfiaktion hängt dabei wesentlich von der Konzentration des Essigsäureesters im Veresterungsgemisch ab und beträgt in der Regel 5 bis 30% des Zulaufs.

Die anfallende Leichtsiederfraktion, die, wie erwähnt, hauptsächlich aus Alkanol 10 bis 50 Gew.-%), Essigsäureester 10 bis 80 Gew.-%) und (Meth)acrylsäureester 1 bis 30 Gew.-%) besteht, wird mit einer 5 bis 40 gew.-%igen Alkalilauge bei Siedetemperatur innerhalb von 1 bis 10 Stunden verseift.

Die Leichtsiederfraktion kann dabei ggf. noch in einem weiteren Destillationsschritt in ein Kopfprodukt, hauptsächlich aus Alkanol und Essigsäureester bestehend, das dann verseift wird, und ein Sumpfprodukt, das im wesentlichen aus (Meth)acrylsäureester besteht, aufgetrennt werden.

Der erhaltene (Meth)acrylsäureester wird dabei vorzugsweise der destillativen Aufarbeitung des Veresterungsgemisches zugeführt.

Die Umsetzung mit Alkalilauge (Verseifung) kann kontinuierlich oder diskontinuierlich, drucklos oder bei Über- oder Unterdruck durchgeführt werden. Vorzugsweise wird dazu ein Rühr- oder Rohrreaktor eingesetzt.

Die Abtrennung des Alkanols aus dem erhaltenen Verseifungsgemisch hängt von der Art des Alkanols, d.h. von dessen Wasserlöslichkeit, ab. In Wasser unlösliche Alkanole bilden eine zweite Phase aus und können einfach abgetrennt werden. In Wasser lösliche Alkanole werden z.B. durch Destillation bzw. durch Strippen mit Luft oder Wasserdampf abgetrennt. Vorzugsweise werden die erhaltenen Alkanole dann wiederum der Veresterung zugeführt. Die destillative Abtrennung oder das Strippen kann beispielsweise in einem beheizbaren Rührreaktor mit einer aufgesetzten Kolonne erfolgen. Der Energieeintrag kann auf herkömmliche Weise erfolgen (Doppelwand-, Rohrschlangenheizung, Umlauferhitzer

Das Strippen des Alkanols in einer Strippkolonne kann auf übliche Weise erfolgen. Beispielsweise kann die heiße (40 bis 80°C) Verseifungslösung am Kopf einer Kolonne eingespeist werden und im Gegenstrom mit Luft (1 bis 20 m³/m³) oder Dampf 0,1 bis 10 t/m³) gestrippt werden. Die Kondensation des Alkanols aus dem Strippgas kann mit einem herkömmlichen Kühler, z.B. einem Rohrbündel oder Plattenwärmetauscher, erfolgen.

Das Alkanol kann dann wieder der Veresterung gemäß Stufe D zugeführt werden.

Das erfindungsgemäße Verfahren weist die folgenden Vorteile auf:
1. Es wird eine äußerst reine (Meth)acrylsäure, d.h. eine (Meth)acrylsäure, die frei beziehungsweise im wesentlichen frei von Aldehyden und weitgehend frei von Essigsäure ist, gewonnen.
2. Die destillative Abtrennung der Essigsäure verläuft ohne Polymerisationsprobleme. Dies ist überraschend, da bekanntlich die Zugabe von aminogruppenhaltigen Verbindungen die Stabilität der (Meth)acrylsäure stark vermindert.
3. Das bei der destillativen Trennung von (Meth)acrylsäure und Essigsäure als Leichtsiederfraktion anfallende Gemisch aus (Meth)acrylsäure und Essigsäure wird ökonomisch sinnvoll verwertet.
4. Die aus der essigsäurehaltigen Fraktion gewonnen (Meth)acrylsäureester sind bereits frei von Aldehyden und müssen deshalb nicht zusätzlich gereinigt werden.
5. Durch die Verseifung des hierin definierten Auftrennungsgemischs wird das im Essigsäureester gebundene Alkanol zurückgewonnen. Die aufgrund der Ausbildung binärer Azeotrope schwierige Trennung des Alkanols vom Essigsäureester, die sonst aus ökonomischen Gründen notwendig gewesen wäre, kann dadurch unterbleiben.

Die vorliegende Erfindung soll nunmehr noch anhand eines Beispiels näher erläutert werden:

### Beispiel

In einem Verweilzeitbehälter wird Roh-Acrylsäure, die durch Propenoxidation analog der DE-A 43 08 087 hergestellt wurde und neben Acrylsäure im wesentlichen 0,1 Gew.-% Diacrylsäure, 0,2 Gew.-% Essigsäure, 0,04 Gew.-% Propionsäure, 0,05 Gew.-% Maleinsäure, 0,06 Gew.-% Furfural, 0,01 Gew.-% Benzaldehyd, 0,08 Gew.-% Wasser und 500 ppm Phenothiazin enthielt mit 0,2 Gew.-% Aminoguanidinhydrogencarbonat versetzt und bei 23°C sechs Stunden lang durch Umpumpen vermischt.

Das so hergestellte Gemisch wurde einer Destillationseinheit zugeführt, die aus einer Dual-Flow-Kolonne mit 25 Böden, einem Robert-Verdampfer und einem Quench-Kondensator bestand. Der Zulauf (11 m³/h) erfolgte auf Boden 22, die Sumpftemperatur betrug 87°C und die Kopftemperatur 68°C bei 60 mbar. Der Quenchkondensator wurde bei 30°C und mit einer Umlaufmenge von 400 m³/h betrieben.

Die Stabilisierung der Kolonne erfolgte mit Thiodiphenylammoniumdodecylbenzolsulfonat, das durch Umsetzung von Phenothiazin (600 ppm) mit Dodecylbenzolsulfonsäure (1000 ppm) in der Quenchflüssigkeit hergestellt wurde, wobei die Quenchflüssigkeit als Rücklauf auf den obersten Kolonnenboden aufgebracht wurde (2 m³/h). Die Quenchflüssigkeit wurde mit 300 ppm Phenothiazin stabilisiert und enthielt neben Acrylsäure im wesentlichen noch 1,9 Gew.-% Essigsäure und 2,1 Gew.-% Wasser. Es wurden 1 m³/h ausgeschleust.

Die Destillationskolonne konnte problemlos 30 Tage lang betrieben werden.

Die Sumpfflüssigkeit, die hauptsächlich aus Acrylsäure bestand, wurde dem Robert-Verdampfer einer zweiten Destillationseinheit zugeführt, deren Kolonne lediglich mit einem Spritzschutz ausgerüstet war und ebenfalls einen Quenchkondensator besaß (400 m³/h Umlaufmenge, T=22°C). Die Quenchflüssigkeit wurde mit 200 ppm Hydrochinonmonomethylether stabilisiert.

Die Sumpftemperatur betrug 73°C, die Kopftemperatur 73°C bei 60 mbar. Das Kondensat (9,3 m³/h) besaß im wesentlichen folgende Zusammensetzung:
99,8 Gew.-% Acrylsäure
0,04 Gew.-% Essigsäure
0,04 Gew.-% Propionsäure, und
0,03 Gew.-% Wasser.

Aldehyde waren gaschromatographisch nicht mehr nachweisbar.

Eine Rührkesselkaskade, bestehend aus drei Rührreaktoren mit je 1 1 Reaktionsvolumen, die mit Kolonne, Kondensator und Phasentrenngefäß ausgerüstet waren, wurde mit 500 g des Quenchaustrages, der im wesentlichen aus 95,9 Gew.-% Acrylsäure, 1,9 Gew.-% Essigsäure und 2.1 Gew.-% Wasser bestand, 550 g Butanol, 0,1 Gew.-% Phenothiazin und 15 g Schwefelsäure pro Stunde beschickt.

Die Reaktionstemperatur in den Rührreaktoren betrug jeweils 103°C, 117°C und 122°C, der Druck betrug 700 mbar.

Am Kopf der Kolonne fiel ein Gemisch aus Wasser, Butanol, Butylacetat und Butylacrylat an, das in ein wäßrige und eine organische Phase zerfiel, wobei die organische Phase, versetzt mit 300 ppm Phenothiazin, der Kolonne als Rücklauf zugeführt wurde.

Der Reaktoraustrag (930 g/h) wurde auf 30°C abgekühlt, die nicht umgesetzte Acrylsäure und der Katalysator mit 5 %-iger Natronlauge neutralisiert, mit Wasser gewaschen und anschließend in einer Siebbodenkolonne mit 60 Böden destilliert.

Der Zulauf zur Kolonne erfolgte auf den 5. Boden, die Sumpftemperatur betrug 109°C, die Kopftemperatur 86°C bei 160 mbar. Am Kopf der Kolonne fielen 910 g/h Destillat an, das sich in eine organische Phase und eine Wasserphase trennte. 806 g/h der mit 300 ppm Phenothiazin versetzten organischen Phase wurden als Rücklauf wieder auf den obersten Boden der Kolonne aufgebracht und 92 g/h ausgeschleust.

Die organische Phase des Destillats enthielt im wesentlichen 20 Gew.-% Essigsäurebutylester, 36,6 Gew.-% Butanol, und 38,0 Gew.-% Butylacrylat.

Aus dem Sumpfablauf wurde in einer weiteren Siebbodenkolonne (30 Böden) das Butylacrylat (738 g/h) als Kopfprodukt isoliert. Die Sumpftemperatur betrug 108°C, die Kopftemperatur betrug 80°C bei 100 mbar und das Rücklaufverhältnis betrug 0,6.

Das gewonnene Butylacrylat besaß eine Reinheit von 99,8 %, enthielt 130 ppm Butylacetat und war aldehydfrei.

In einem Rührreaktor wurde ein Gemisch aus 1000 g der organischen Phase des Destillats mit 30 %-iger Natronlauge (2000 g) zwei Stunden lang unter Rückfhiß erhitzt. Nach Beendigung der Verseifungsreaktion wurde das gebildete Butanol unter Vakuum (500 mbar) über eine Kolonne (10 Glockenböden) aus dem Reaktor destillativ abgetrennt. Das Kondensat zerfiel in eine Wasserphase und eine organische Phase (706 g). Die organische Phase enthielt ungefähr 84 Gew.-% Butanol und kann, mit frischem Butanol vermischt, direkt wieder der Veresterung zugeführt werden.

Es konnten auf diese Weise 79 % der theoretischen Butanolmenge zurückgewonnen werden.

## Patentansprüche

1. Verfahren zur Herstellung von Rein-(Meth)acrylsäure, ausgehend von einer Roh-(Meth)äcrylsäure, die mindestens 90 Gew.-% (Meth)acrylsäure und neben (Meth)acrylsäure noch 0,2 bis 10 Gew.-% Essigsäure, 0,05 bis 1 Gew.-% Aldehyde und 0,05 bis 5 Gew.-% Wasser enthält, das die folgenden Stufen umfasst:
A: Behandlung der Roh-(Meth)acrylsäüre mit mindestens einer Verbindung, die in der Lage ist, Aldehyde zu entfernen, wobei eine aldehydfreie Roh-(Meth)acrylsäure erhalten wird;
B: Unterwerfen der aldehydfreien Roh-(Meth)acrylsäure einer unscharfen Destillation, wobei eine (Meth)acrylsäure und Essigsäure enthaltende Leichtsiederfraktion *mit mindestens 80 Gew.-% (Meth)acrylsäure,* und eine (Meth)acrylsäure und Hochsieder enthaltende Schwersiederfraktion erhalten wird; und
C: Abtrennung einer Rein-(Meth)acrylsäure aus der Schwersiederfraktion.
D: Veresterung der in Stufe B erhaltenen, (Meth)acrylsäure und Essigsäure enthaltenden Leichtsiederfraktion mittels einem oder mehreren Alkanolen, wobei ein Veresterungsgemisch umfassend einen oder mehrere (Meth)acrylsäureester, einen oder mehrere Essigsäureester und ein oder mehrere Alkanole erhalten wird.

2. Verfahren nach Anspruch 1, **dadurch gekennzeichnet, dass** die Verbindung, die in der Lage ist, Aldehyde zu entfernen, eine amingruppenhaltige Verbindung ist.

3. Verfahren nach Anspruch 1 oder 2, **dadurch gekennzeichnet, dass** die Stufe B in einer Dual-Flow-Kolonne bei einer Temperatur im Bereich von 60 bis 100°C und einem Druck am Kolonnenkopf von 20 bis 200 mbar sowie einem Rücklaufverhältnis von 1 bis 10 durchgeführt wird.

4. Verfahren nach einem der Ansprüche 1 bis 3, **dadurch gekennzeichnet, dass** die Abtrennung der Rein-(Meth)acrylsäure aus der Schwersiederfraktion durch "Abtoppen" erfolgt.

5. Verfahren nach Anspruch 1, **dadurch gekennzeichnet, dass** es eine weitere Stufe E umfasst:
E: Auftrennung des Veresterungsgemisches unter Erhalt eines oder mehrerer (Meth)acrylsäureester, sowie eines Auftrennungsgemisches, das einen oder mehrere Essigsäureester und ein oder mehrere Alkanole umfasst.

6. Verfahren nach Anspruch 5, **dadurch gekennzeichnet, dass** es eine zusätzliche Stufe F umfasst:
F: Verseifung des Auftrennungsgemisches, wobei ein Verseifungsgemisch, das ein oder mehrere Alkanole und Essigsäuresalze enthält, erhalten wird.

7. Verfahren nach Anspruch 6, **dadurch gekennzeichnet, dass** das oder die Alkanole aus dem Verseifungsgemisch abgetrennt werden und in die Veresterung gemäß Stufe D zurückgeführt wird.

8. Verfahren nach Anspruch 1, **dadurch gekennzeichnet, daß** in Stufe B eine Sulfonsäuresalz des Phenothiazins als Polymerisationsinhibitor für (Meth)acrylsäure, eingesetzt wird.

## Claims

1. A process for preparing pure (meth)acrylic acid starting from a crude (meth)acrylic acid which contains at least 90% by weight (meth)acrylic acid, and in addition to (meth)acrylic acid also from 0.2 to 10% by weight acetic acid, from 0.05 to 1% by weight aldehydes and from 0.05 to 5% by weight water, which comprises the following stages:
A: treating the crude (meth)acrylic acid with at least one compound which is able to remove aldehydes, to give an aldehyde-free crude (meth)acrylic acid;
B: subjecting the aldehyde-free crude (meth)acrylic acid to imprecise distillation, to give a low-boiling fraction comprising (meth)acrylic acid and acetic acid with at least 80% by weight (meth)acrylic acid and a high-boiling fraction comprising (meth)acrylic acid and high boilers; and
C: isolating a pure (meth)acrylic acid from the high-boiling fraction;
D: esterifying the low-boiling fraction comprising (meth)acrylic acid and acetic acid that is obtained in stage B by means of one or more alkanols, to give an esterification mixture comprising one or more (meth)acrylates, one or more acetic esters, and one or more alkanols.

2. A process as claimed in claim 1, wherein the compound which is able to remove aldehydes is an amino-functional compound.

3. A process as claimed in claim 1 or 2, wherein stage B is conducted in a dual-flow column at a temperature in the range from 60 to 100°C and at a column-top pressure of from 20 to 200 mbar and with a reflux ratio of from 1 to 10.

4. A process as claimed in any one of claims 1 to 3, wherein the pure (meth)acrylic acid from the high-boiling fraction is separated off by "topping".

5. A process as claimed in claim 1, which comprises a further stage E:
E: separating the esterification mixture to give one or more (meth)acrylates and a separation mixture which comprises one or more acetic esters and one or more alkanols.

6. A process as claimed in claim 5, which comprises an additional stage F:
F: hydrolyzing the separation mixture to give a hydrolysis mixture which comprises one or more alkanols and acetic salts.

7. A process as claimed in claim 6, wherein the alkanol or alkanols is or are separated off from the hydrolysis mixture and passed back to the esterification of stage D.

8. A process as claimed in claim 7, wherein a sulfonic salt of phenothiazine is used in stage B as a polymerization inhibitor for (meth)acrylic acid.

## Revendications

1. Procédé de préparation d'acide (méth)acrylique pur à partir d'un acide (méth)acrylique brut contenant au moins 90% en poids d'acide (méth)acrylique et, outre de l'acide (méth)acrylique, de 0,2 à 10% en poids d'acide acétique, de 0,05 à 1 % en poids d'aldéhydes et de 0,05 à 5% en poids d'eau, et comprenant les étapes suivantes:
A: traitement de l'acide (méth)acrylique par au moins un composé capable d'éliminer les aldéhydes, avec obtention d'acide (méth)acrytique brut exempt d'aldéhydes,
B: envoi de l'acide (méth)acrylique brut à une distillation grossière, par laquelle on obtient une fraction à bas point d'ébullition contenant de l'acide (méth)acrylique et de l'acide acétique avec au moins 80% en poids d'acide (méth)acrylique, et une fraction à haut point d'ébullition contenant de l'acide (méth)acrylique et des constituants lourds, et
C: séparation d'un acide (méth)acrylique pur de la fraction à haut point d'ébullition,
D: estérification de la fraction à bas point d'ébullition obtenue à l'étape B et contenant l'acide (méth)acrylique et de l'acide acétique au moyen d'un ou plusieurs alcanols, avec obtention d'un mélange d'estérification comprenant un ou plusieurs esters d'acide (méth)acrylique, un ou plusieurs esters d'acide acétique et un ou plusieurs alcanols.

2. Procédé selon la revendication 1, **caractérisé en ce que** le composé capable d'éliminer les aldéhydes est un composé contenant des groupes amine.

3. Procédé selon la revendication 1 ou 2, **caractérisé en ce que** l'étape B est entreprise dans une colonne à plateaux dualflow à une température de l'ordre de 60 à 100°C et une pression en tête de colonne de 20 à 200 mbar, et une proportion de reflux de 1 à 10.

4. Procédé selon l'une quelconque des revendications 1 à 3, **caractérisé en ce que** la séparation de l'acide (méth)acrylique pur de la fraction à haut point d'ébullition est réalisée par "séparation de volatils".

5. Procédé selon la revendication 1, **caractérisé en ce qu'**il comprend une autre étape E:
E: séparation du mélange d'estérification avec obtention d'un ou plusieurs esters d'acide (méth)acrylique, ainsi que d'un mélange de séparation comprenant le ou les esters d'acide acétique et un ou plusieurs alcanols.

6. Procédé selon la revendication 5, **caractérisé en ce qu'**il comprend une étape additionnelle F:
F: saponification du mélange de séparation, avec obtention d'un mélange de saponification contenant un ou plusieurs alcanols et sels d'acide acétique.

7. Procédé selon la revendication 6, **caractérisé en ce que** le ou les alcanols sont séparés du mélange de saponification et sont recyclés dans l'estérification selon l'étape D.

8. Procédé selon la revendication 1, **caractérisé en ce que**, dans l'étape B, on met en oeuvre de la phénothiazine en tant qu'inhibiteur de polymérisation pour l'acide (méth)acrylique.
